**Europäisches Patentamt**

**European Patent Office**

**Office européen des brevets**

(19)

(11) Veröffentlichungsnummer : **0 141 338**
**B1**

(12) **EUROPÄISCHE PATENTSCHRIFT**

(45) Veröffentlichungstag der Patentschrift :
11.01.89

(21) Anmeldenummer : 84112507.3

(22) Anmeldetag : 17.10.84

(51) Int. Cl.⁴ : **B 65 C   9/18**, B 65 C   9/30,
B 65 C   9/36, B 65 C   1/02,
A 41 B 13/02

(54) **Vorrichtung zum Abtrennen und Aufbringen von Streifenabschnitten auf im Abstand hintereinander liegende Bereiche einer Materialbahn.**

(30) Priorität : 21.10.83 DE 3338306
30.08.84 DE 3431910

(43) Veröffentlichungstag der Anmeldung :
15.05.85 Patentblatt 85/20

(45) Bekanntmachung des Hinweises auf die Patenterteilung : 11.01.89 Patentblatt 89/02

(84) Benannte Vertragsstaaten :
BE DE FR IT LU NL

(56) Entgegenhaltungen :
DE--B-- 3 009 418
FR--A-- 2 203 358
US--A-- 3 963 557
US--A-- 4 297 157

(73) Patentinhaber : **Paul Hartmann Aktiengesellschaft**
**Paul-Hartmann-Strasse**
**D-7920 Heidenheim (DE)**

(72) Erfinder : **Eschler, Dieter**
**Hellensteinstrasse 27**
**D-7920 Heidenheim (DE)**

(74) Vertreter : **Becker, Maria, Dipl.-Phys.**
**Auf dem Haigst 29**
**D-7000 Stuttgart 70 (DE)**

**Beschreibung**

Die Erfindung betrifft eine Vorrichtung zum Aufbringen von gedehnten Streifenabschnitten einer ersten elastischen Materialbahn — auf diskrete, in einem vorbestimmten Abstand hintereinanderliegende Bereiche einer zweiten, sich kontinuierlich bewegenden Materialbahn gemäß Oberbegriff des Anspruchs 1.

Eine solche Vorrichtung ist aus der US-PS 3,963,557 bekannt. Sie dient dazu, von einem kontinuierlich mit gleicher Geschwindigkeit zugeführten Endlosband abgetrennte Abschnitte definierter Länge in Abständen zueinander auf einem zweiten, ebenfalls kontinuierlich mit gleicher Geschwindigkeit zugeführten Band klebend aufzubringen.

Bei der bekannten Vorrichtung wird dies dadurch erreicht, daß von dem ersten Bandmaterial in definierter Länge abgetrennte Streifenabschnitte von Förderelementen aufgenommen und auf die zweite Bahn übertragen werden, deren Fördergeschwindigkeit nach der Abtrennung eines Streifenabschnittes von der ersten Materialbahn bis zur Erreichung eines vorbestimmten Abstandes zu dem nachfolgenden Streifenabschnitt zunimmt. Nach Übergabe des Streifenabschnittes auf die zweite Materialbahn geht die Fördergeschwindigkeit des betreffenden Förderelementes wieder bis auf den geringeren Wert zurück, bei dem wieder ein neuer Streifenabschnitt von der ersten Materialbahn zur Übertragung auf die zweite Materialbahn übernommen wird.

Bei der aus der US-PS 3,963,557 bekannten Vorrichtung rotieren die Förderelemente mit unterschiedlicher Winkelgeschwindigkeit um eine Achse. Die unterschiedliche Winkelgeschwindigkeit wird dadurch erreicht, daß die auf der Achse rotierenden Förderelemente durch eine Antriebsscheibe angetrieben werden, die auf einer parallel versetzten Achse rotiert und radiale Führungsnuten aufweist, in die an den Förderelementen angebrachte Mitnehmer zur Kraftübertragung eingreifen. Durch eine solche exzentrische Lagerung der Antriebsscheibe und der rotierenden Förderelemente wandern die Mitnehmer der Förderelemente während einer Umdrehung eines Förderelementes innerhalb der Führungsnut der Antriebsscheibe von einem radial minimalen Abstand zur Rotationsachse der Antriebsscheibe bis zu einem radial maximalen größeren Abstand. Diese in radialer Richtung variierende Angriffspunktverschiebung zwischen Antriebsachse und Förderelement bewirkt die gewünschte unterschiedliche Winkelgeschwindigkeit der Förderelemente bei deren Rotation.

Bei dem Verfahren mit jener Vorrichtung variiert die Winkelgeschwindigkeit zwangsläufig über den gesamten Umfangsbereich, d. h. auf dem gesamten Umfang gibt es keinen Punkt, an dem die Förderelemente nicht ihre Geschwindigkeit verändern. Dies ist bei dem Aufbringen von von einem ersten Band abgetrennten Abschnitten, die z. B. mit Kleber versehen auf eine zweite kontinuierlich mit gleichbleibender Geschwindigkeit herangeführte Bahn klebend aufgetragen werden sollen, von Nachteil. Denn die sich stets verändernde Geschwindigkeit, mit der die aufzutragenden Abschnitte befördert werden, verhindert, daß die auf die zweite Bahn aufzutragenden Abschnitte ohne Relativbewegung zwischen dem aufzutragenden Abschnitt und der zweiten Bahn übertragen werden können. Ein Übertragen bei fehlender Relativgeschwindigkeit, d. h. gleicher Geschwindigkeit des aufzutragenden Abschnittes und der zweiten Bahn, ist zur Erzielung einwandfreier Haftbindung der Abschnitte mit der zweiten Bahn jedoch häufig unbedingt erforderlich, und zwar insbesondere dann, wenn ein Kontaktkleber verwendet wird. Dabei ist zu berücksichtigen, daß in der Praxis das Auftragen mit extrem hoher Geschwindigkeit erfolgt und daher die Kontaktzeiten zwangsläufig äußerst kurz sind. Bei stets vorhandener Relativgeschwindigkeit zwischen dem zweiten Band und den Förderelementen ist praktisch nur das Auftragen von relativ kurzen Abschnitten auf das zweite Band möglich. Bei längeren aufzutragenden Abschnitten käme es dagegen während des Auftragsvorgangs zu unzulässigen Spannungen zwischen dem aufzutragenden Abschnitt und dem zweiten Band.

Aus der US-PS 4,297,157 sind überdies ein Verfahren und eine Vorrichtung bekannt, mit denen sich Streifen einer ersten Materialbahn auf Bereiche einer zweiten Materialbahn übertragen lassen. Auch hier ist die Länge der Bereiche der zweiten Materialbahn größer als die Länge der aufzubringenden Streifen. Bei diesem Verfahren wird die Streifenlänge dadurch eingestellt, daß die von der ersten Materialbahn abgenommenen Streifen von gegeneinander beweglichen Klemmeinrichtungen gehalten werden und der Abstand dieser Einrichtungen nach Aufnahme der Streifenabschnitte vermindert wird, bis die gewünschte Länge erreicht ist. Ein wesentlicher Nachteil dieses Verfahrens ist es, daß die Streifen über die beim Übertragen auf die zweite Materialbahn notwendige Länge hinaus gedehnt werden und dadurch aufgrund der Hysterese des Materials einen irreversiblen Spannungsverlust erleiden.

Es ist daher Aufgabe der Erfindung, eine Vorrichtung zu schaffen, mit der sich eine optimale Verbindung von Streifen einer ersten Materialbahn mit Bereichen einer zweiten Materialbahn herstellen läßt.

Diese Aufgabe wird bei einer Vorrichtung der eingangs genannten Art mit Hilfe der in Anspruch 1 genannten Merkmale gelöst. Dadurch, daß der Streifen der ersten Materialbahn bei der Übertragung auf die zweite Materialbahn mit einer konstanten Geschwindigkeit bewegt wird, die der der zweiten Materialbahn entspricht, ist gewährleistet, daß auch bei den hohen Umlaufgeschwindigkeiten der die Streifenabschnitte haltenden Förderelemente eine optimale Klebeverbindung zwischen Streifen und zweiter Materialbahn erzielt wird.

Die Größe der Winkelbereiche, über die sich die einzelnen Kurvenabschnitte erstrecken, ist in großen Grenzen frei wählbar.

Vorteilhafterweise nimmt der Kurvenabschnitt a, in dem die Hebel der Vorderelemente während der Übernahme der zweiten Materialbahn und deren Verbindung mit den von der ersten Bahn abgetrennten Abschnitten geführt sind, einen Winkelbereich von mindestens 150° bis 180° ein. Auf diese Weise kann die kontinuierlich mit konstanter Geschwindigkeit zuzuführende zweite Bahn über einen relativ großen Winkelbereich in Kontakt mit demjenigen Förderelement geführt werden, von dem der von der ersten Bahn abgetrennte und mit einem Klebstoff versehene Streifenabschnitt auf den jeweils in Frage kommenden Bereich der zweiten Materialbahn übertragen wird. Voraussetzung für ein über einen größeren Winkelbereich erfolgendes Kontaktieren zwischen einem Förderelement und der zweiten Materialbahn, insbesondere wenn diese aus einem nicht-dehnbaren Material besteht, ist eine konstante Winkelgeschwindigkeit des Förderelementes während der gesamten Kontaktzeit, in der die Fördergeschwindigkeit der zweiten Materialbahn mit der Umfangsgeschwindigkeit des betreffenden Förderelementes exakt übereinstimmen muß. Andernfalls wäre eine konstante Zuführgeschwindigkeit der zweiten Bahn nicht möglich.

Die auf minimalem Abstand aneinander geführten Förderelemente werden während eines vorbestimmbaren Zeitraumes, in dem das Abtrennen der von den Förderelementen aufgenommenen Abschnitte der ersten Bahn erfolgt, durch gleichzeitiges Führen des in Rotationsrichtung vorderen Förderelementes auf dem Kurvenabschnitt c und des nachfolgenden Förderelementes auf dem Kurvenabschnitt a auf gleicher Winkelgeschwindigkeit gehalten. Diese gleiche Winkelgeschwindigkeit ergibt sich aus dem Umstand, daß beide Kurvenabschnitte a und c konzentrisch zur Antriebsachse verlaufende Kreisabschnitte sind. Der Winkelbereich, auf dem die beiden benachbarten Förderelemente gemeinsam auf jeweils einem konzentrischen Kreisbogenabschnitt geführt werden, ist in bestimmten Grenzen frei wählbar.

Eine besondere Ausgestaltung der Klemmeinrichtung an den einzelnen Förderelementen zum Aufnehmen und Übertragen der Abschnitte der ersten Bahn ist Gegenstand des Patentanspruchs 4.

Ein Ausführungsbeispiel ist in der Zeichnung dargestellt. Es zeigen :

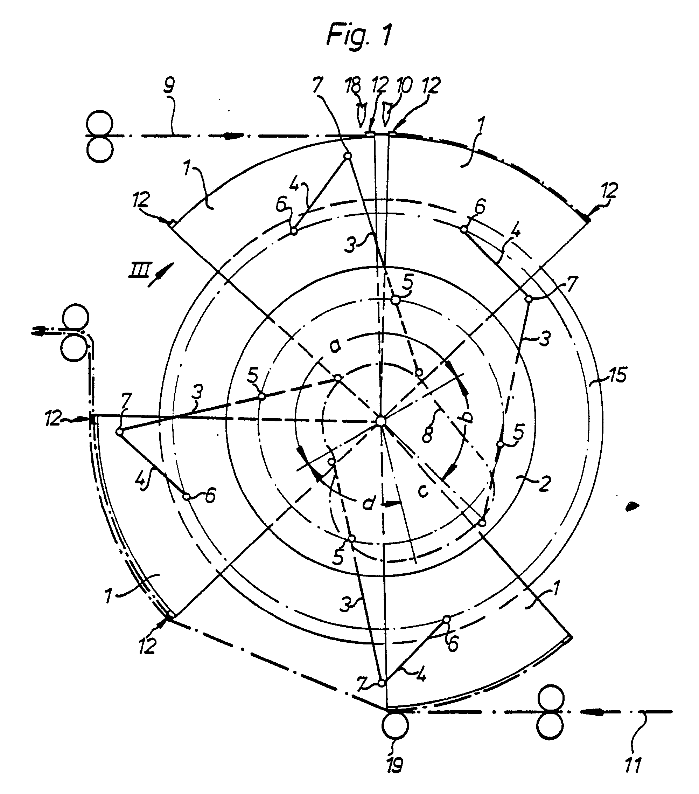

Fig. 1 den schematischen Aufbau der Vorrichtung in Form einer Ansicht,

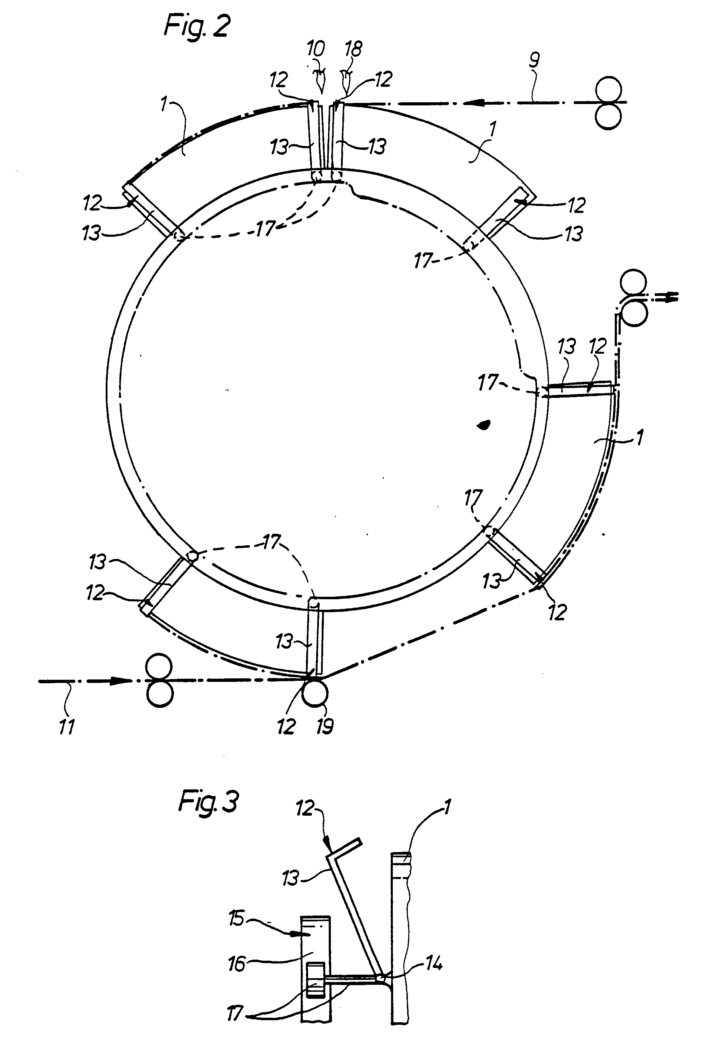

Fig. 2 die in schematischer Form in Fig. 1 dargestellte Vorrichtung in einer Ansicht von hinten,

Fig. 3 einen Teil der an den Förderelementen angreifenden Klemmeinrichtung nach der in Fig. 1 mit Pfeil III angegebenen Ansicht in Form eines Ausschnittes.

In der Aufbringvorrichtung rotieren die Förderelemente 1 je Umdrehung mit unterschiedlicher Winkelgeschwindigkeit um die Mittelachse. Der Antrieb der Förderelemente 1 erfolgt über eine Antriebsscheibe 2, die um die gleiche Achse rotiert wie die Förderelemente 1. Mit der Antriebsscheibe 2 sind die einzelnen Förderelemente jeweils über einen ersten Hebel 3 sowie einen zweiten Hebel 4 verbunden. An der Antriebsscheibe 2 selbst sind die ersten Hebel auf einem gleichen Teilkreis in zur Mittelachse parallelen Achsen 5 drehbar gelagert. Die zweiten Hebel sind jeweils an einem bei jedem Förderelement 1 an der gleichen Stelle liegenden Punkt 6 in gleicher Weise drehbar gelagert wie die ersten Hebel 3 in den Achsen 5. Die ersten Hebel 3 und die zweiten Hebel 4 sind jeweils in Gelenken 7 miteinander verbunden. Die noch verbleibenden freien Enden der ersten Hebel sind auf einer Kurvenbahn 8 zwangsgeführt. Die Form der Kurvenbahn 8 ist so ausgelegt, daß sich die Winkelgeschwindigkeit der Förderelemente 1 auf einer Umdrehung verändert.

Zu diesem Zweck weist die Kurvenscheibe 8 Abschnitte a-d auf. Davon erstreckt sich der Abschnitt a über einen Winkelbereich von 180° und der Abschnitt c über etwa 30°. Diese beiden Abschnitte sind ferner konzentrisch um die Antriebsachse verlaufende Kreisbögen. Da auch die Anlenkpunkte 5 und 6 der mit den Förderelementen verbundenen Hebel 3 und 4 auf zur Antriebsachse konzentrischen Kreisbahnen geführt werden, weisen die Förderelemente 1 bei Führung auf den Kurvenabschnitten a und c jeweils konstante, mit der Antriebsscheibe 2 übereinstimmende Winkelgeschwindigkeiten auf. Auf den Abschnitten b und d erfolgt dagegen eine Verzögerung bzw. eine Beschleunigung der dort jeweils geführten Förderelemente 1.

Eine in Streifenabschnitte aufzutrennende erste Materialbahn 9 wird der Aufbringvorrichtung in einem Bereich zugeführt, in dem zwei Förderelemente 1 auf minimalen, äußerst geringen Abstand aneinandergeführt sind. Nach Erreichen des minimalen Abstandes wird die erste Materialbahn 9 an dem in Rotationsrichtung vorderen Ende des in Rotationsrichtung hinteren Förderelements durch Schließen der Klemmvorrichtung 12 fixiert. Etwa gleichzeitig fixiert die Klemmvorrichtung 12 des hinteren Endes des vorderen Förderelementes 1 dort das noch freie Ende des auf dem vorderen Förderelement 1 sich bereits befindlichen Teils der Materialbahn 9. Sodann bewegen sich die beiden benachbarten Förderelemente 1 durch gleichzeitige Führung auf den Kurvenabschnitten a (hinteres Förderelement) und c (vorderes Förderelement) über einen Winkelbereich von ca. 30° mit gleicher Geschwindigkeit. In dieser Phase trennt ein mitlaufendes Messer 10 einen ersten, auf dem vorderen Förderelement liegenden Streifenabschnitt ab.

Die erste Materialbahn 9 kann aus elastischem Material sein, das so zugeführt wird, daß es auf dem aufnehmenden Förderelement in gedehntem Zustand zwischen den dort vorgesehenen Klemmvorrichtungen 12 aufliegt. Beim Übertragen auf das Förderelement wird gleichzeitig durch eine Klebstoffauftragvorrichtung 18 der zwischen den Klemmvorrichtungen 12 liegende Bereich der ersten Materialbahn 9 mit Klebstoff beschichtet.

Nach erfolgter Abtrennung eines Streifenabschnittes durch das Messer 10 beschleunigt das vordere der benachbarten Förderelemente 1 durch Führung auf dem Kurvenabschnitt d. Das nachfolgende Förderelement verlangsamt dagegen seine Geschwindigkeit durch Führung auf dem Kurvenabschnitt B.

Die Phase des Auseinanderlaufens dauert so lange an, bis die beiden Förderelemente zwischen ihren Aufnahmeflächen für die Streifenabschnitte der ersten Materialbahn 9 einen Abstand aufweisen, der demjenigen entspricht, der auf der zweiten Materialbahn 11, auf die die Streifenabschnitte aufzubringen sind, zwischen den aufgebrachten Streifenabschnitten gewünscht ist.

Wenn dieser Abstand erreicht ist, wird die Führung des jeweils vorderen Förderelements von dem Kurvenabschnitt a übernommen, d. h. das Förderelement bewegt sich fortan über die gesamte Länge dieses Kurvenabschnitts mit konstanter, der Antriebsscheibe 2 entsprechender Winkelgeschwindigkeit. In diesem Führungsbereich wird die zweite Materialbahn 11 den Förderelementen mit einer der Umfangsgeschwindigkeit der Förderelemente in diesem Bereich entsprechenden Geschwindigkeit kontinuierlich zugeführt und über eine Andrückrolle 19 mit der Klebstoffschicht der Streifenabschnitte der ersten Materialbahn 9 kontaktiert. Abgeführt wird die zweite Materialbahn 11 von der Umfangsbahn der Förderelemente 1 erst etwa 90° nach der ersten Kontaktstelle an der Andrückrolle 19.

In der Möglichkeit der Erzielung einer derart langen Kontaktzeit zwischen der zweiten Materialbahn 11 und den auf den Förderelementen 1 in gedehntem Zustand fixierten Streifenabschnitten der ersten Materialbahn 9 liegt ein ganz wesentlicher Vorteil der erfindungsgemäß aufgebauten Aufbringvorrichtung. Die Länge der Kontaktzeit ist dabei in großen Grenzen durch entsprechende Auslegung der einzelnen in keinem festen Verhältnis zueinander stehenden Kurvenabschnitte a-d sowie der Festlegung der Zu- und Abführstellen für die zweite Materialbahn 11 frei wählbar. Die Klemmvorrichtungen 12 geben die fixierten gedehnten Streifenabschnitte auf den Förderelementen 1 erst kurz vor Ablenkung der zweiten Materialbahn 11 aus der Umlaufbahn der Förderelemente frei.

Nach Freigabe der Streifenabschnitte werden die Förderelemente in dem an den Kurvenabschnitt b angrenzenden Endbereich des Kurvenabschnitts a wieder zur Aufnahme eines weiteren Streifenabschnittes der ersten Materialbahn 9 an das vorauslaufende, seine Geschwindigkeit verzögernde Förderelement 1 herangeführt, und ein neuer Übertragungszyklus beginnt.

Die vorstehend erwähnten Klemmvorrichtungen 12 bestehen aus einem schwenkbaren Winkelhebel 13, von dem ein Schenkel an die Aufnahmefläche des jeweiligen Förderelementes 1 andrückbar ist. Das Schwenken erfolgt um mit den Förderelementen jeweils fest verbundene Achsen 14. Bei geöffneter Klemmvorrichtung 12 ist der kurze Schenkel des Winkelhebels 13 vollständig aus dem Bereich der Aufnahmefläche des Förderelementes 1 herausgeschwenkt. Die Steuerung der Schwenkbewegung um die Achsen 14 erfolgt durch eine Kurvenscheibe 15. In dieser Kurvenscheibe 15 ist eine Steuerkurve 16 vorgesehen, in die von den Winkelhebeln 13 ein fest mit den Winkelhebeln verbundener Steuerstift 17 eingreift.

Die Aufnahmeflächen der Förderelemente für die Streifenabschnitte der ersten Materialbahn 9 können selbstverständlich auch mit jeder anderen Art von Fixiereinrichtungen, wie z. B. Saugbohrungen, versehen sein. Andererseits muß auch die Steuerung der hier vorgesehenen Klemmvorrichtungen 12 nicht über eine Kurvenscheibe erfolgen, sondern kann ebenso gut hydraulisch verwirklicht werden.

Das erfindungsgemäße Verfahren und die dazu beispielhaft beschriebene Vorrichtung eignen sich insbesondere dazu, bei Windeln, z. B. im Bereich der Beinabschnitte, elastisches Band in gedehntem Zustand lediglich auf den Bereich begrenzt aufzubringen, in dem der zu raffende Beinabschnitt der Windeln liegt. Bei den Windeln handelt es sich dabei bevorzugt um Wegwerfwindeln, die durch Abtrennen von einer aus mehreren Bestandteilen bestehenden Materialbahn gewonnen werden. Bei der Herstellung solcher Windeln ist es mit dem erfindungsgemäßen Verfahren und der dazu angegebenen Vorrichtung möglich, das elastische in den Beinbereichen aufzubringende Band kontinuierlich von einer Endlosrolle aufzubringen und dennoch die einzelnen Streifenabschnitte mit Abstand zueinander auf die sich bewegende Windelmaterialbahn unter Einhaltung ausreichend langer Kontaktzeiten aufzubringen.

**Patentansprüche**

1. Vorrichtung zum Aufbringen von gedehnten Streifenabschnitten einer ersten elastischen Materialbahn (9) auf diskrete, in einem vorbestimmten Abstand hintereinander liegende Bereiche einer zweiten, sich kontinuierlich bewegenden Materialbahn (11), bei der eine Vielzahl von Förderelementen (1) mit jeweils einer an beiden hintereinander liegenden Materialbahnen anlegbaren, auf einem gleichen Kreisumfang liegenden und umlaufenden Aufnahmefläche auf einer gemeinsamen Achse rotiert und bei der die Förderelemente (1) umfangsmässig Abstand zueinander aufweisen und je Umdrehung ihre Winkelgeschwindigkeit nach einer bestimmten Vorgabe derart variieren, dass in einer ersten fest vorgegebenen Position, in die die erste Materialbahn (9) auf die Aufnahmeflächen der Förderelemente (1) aufgetragen wird, jeweils zwei Förderelemente mit minimalem Abstand hintereinander liegen, aus der sich das in Bewegungsrichtung vordere Förderelement mit gegenüber dem benachbarten nachfolgenden Förderelement (1) höherer Winkelgeschwindigkeit unter Abstandsvergrösserung entfernt und nach Erreichen eines Abstandes, in dem die mitgeführten Streifenabschnitte auf die zweite Materialbahn (11) aufzubringen sind, die Streifenabschnitte in einer zweiten fest vorgegebenen Position auf die zweite Materialbahn (11) überträgt, gekennzeichnet durch die Merkmale :

a) Auf der Achse der Förderelemente (1) rotiert eine Antriebsscheibe (2).

b) Auf einem Teilkreis der Antriebsscheibe (2) ist je Förderelement (1) ein erster Hebel (3) in einer zur gemeinsamen Achse der Förderelemente (1) parallelen Achse (5) drehbar gelagert.

c) Das radial innen liegende Ende jedes ersten Hebels (3) ist in einer vorgegebenen Kurvenbahn (8) führbar.

d) An jedem radial aussen liegenden Ende jedes ersten Hebels ist ein zweiter Hebel (4) angelenkt.

e) Die zweiten Hebel (4) sind mit jeweils einem Förderelement (1) gelenkig verbunden.

f) Die Anlenkpunkte (6) für die zweiten Hebel (4) liegen an jedem Förderelement (1) an der gleichen Stelle.

g) Die Kurvenbahn (8) ist in vier aufeinanderfolgende Abschnitte (a, b, c, d) aufgeteilt, von denen zwei im wesentlichen gegenüberliegende Abschnitte (a, c) zur gemeinsamen Achse der Förderelemente (1) konzentrische Kreisbögen sind, wobei einer als Kurvenbahn (b) mit zur gemeinsamen Achse zunehmendem Radius und einer als Kurvenbahn (d) mit zur gemeinsamen Achse abnehmendem Radius ausgebildet ist.

h) Der erste Kurvenabschnitt (a), in dem die Führung der Förderelemente (1) während der Übernahme der zweiten Materialbahn (11) und deren Verbindung mit den Streifenabschnitten der ersten Materialbahn (9) erfolgt, erstreckt sich über einen Winkelbereich, der derart gewählt ist, dass jedes Förderelement über eine mindestens der Länge der Streifenabschnitte entsprechende Entfernung mit konstanter Winkelgeschwindigkeit bewegt wird, um dabei einen Streifenabschnitt auf die zweite Materialbahn (11) aufzubringen, wobei die Geschwindigkeit der Förderelemente (1) mit der der zweiten Materialbahn (11) übereinstimmt.

2. Vorrichtung nach Anspruch 1, dadurch gekennzeichnet, dass der erste Kurvenabschnitt (a) zur Führung der Förderelemente (1) während der Übernahme der zweiten Materialbahn (11) und deren Verbindung mit den Streifenabschnitten der ersten Materialbahn (9) dient und sich über einen Winkelbereich von mindestens 150° bis etwa 180° erstreckt.

3. Vorrichtung nach Anspruch 1 oder 2, dadurch gekennzeichnet, dass während des Abtrennens der Streifenabschnitte von der ersten Materialbahn durch ein Messer (10) die benachbarten Förderelemente minimalen Abstand haben, wobei während eines vorbestimmbaren gleichen Zeitraums das in Rotationsrichtung vordere Förderelement (1) auf dem Kurvenabschnitt (c) und das benachbarte nachfolgende Förderelement (1) auf dem ersten Kurvenabschnitt (a) geführt ist.

4. Vorrichtung nach einem der Ansprüche 1 bis 3, bei der am umfangmässigen Anfang und Ende der Aufnahmefläche eines Förderelements (1) je eine Klemmvorrichtung (12) angreift, die bei bestimmten Winkelstellungen der Förderelemente (1) geschlossen oder geöffnet ist, dadurch gekennzeichnet, dass die Klemmvorrichtung (12) ein um eine zur kreisbogenförmigen Aufnahmefläche der Förderelemente parallele Achse schwenkbarer Winkelhebel (13) ist, dessen einer Schenkel an die Aufnahmefläche des Förderelements (1) andrückbar ist und dessen anderer Schenkel durch die Führung über einen Steuerstift (17) an einer Kurvenscheibe (15) alternierend in die Öffnungs- und Schließstellung schwenkbar ist.

5. Verwendung einer Vorrichtung nach einem der Ansprüche 1 bis 4 zur Herstellung von aus Bahnmaterialabschnitten bestehenden Wegwerfwindeln mit in Längsrichtung der Bahnmaterialabschnitte verlaufenden Beinabschlussbereichen, die nur eine zwischen den Enden der Bahnmaterialabschnitte liegende Teillänge erfassen.

## Claims

1. Device for applying stretched tags of a first elastic web of material (9) to discrete regions lying at a predetermined distance one behind the other on a continuously moving second web of material (11), wherein a plurality of conveying elements (1) rotates on a common axle, each having a receiving surface which lies and rotates on an identical circular circumference and can be placed against the two webs of material one of which is arranged behind the other, and wherein the conveying elements (1) are circumferentially spaced and vary their angular velocity per rotation in such a specified way that two conveying elements are arranged with one at a minimal distance behind the other in a first predetermined position in which the first web of material (9) is placed on the receiving surfaces of the conveying elements (1), and the front conveying element in terms of the direction of movement moves out of this position at a higher angular velocity than the neighbouring conveying element (1) following it, thereby increasing the spacing between them, and, after a spacing has been reached at which the tags which have been taken along are to be applied to the second web of material (11), transfers the tags in a second predetermined position to the second web of material (11), characterized by the features :

a) A driving disc (2) rotates on the axle of the conveying elements (1).

b) On a graduated circle of the driving disc (2), one first lever (3) per conveying element (1) is rotatably mounted in an axle (5) parallel to the common axle of the conveying elements (1).

c) The radially inner end of each first lever (3) is guidable on a predetermined curved path (8).

d) A second lever (4) is articulatedly connected to each radially outer end of each first lever.

e) The second levers (4) are each articulatedly connected to one conveying element (1).

f) The points of articulation (6) for the second levers (4) lie at the same place on each conveying element (1).

g) The curved path (8) is divided up into four successive sections (a, b, c, d), two generally oppositely arranged sections (a, c) of which are arcs of a circle which are concentric with the common axle of the conveying element (1), with one of the sections being designed as a curved path (b) with a radius increasing from the common axle and one as a curved path (d) with a radius decreasing from the common axle.

h) The first curved section (a) in which the conveying elements (1) are guided during takeover of the second web of material (11) and its connection with the tags of the first web of material (9) extends over an angular area which is selected such that each conveying element is moved at a constant angular velocity over a distance corresponding to at least the length of the tags, thereby to apply a tag to the second web of material (11), with the velocity of the conveying elements (1) corresponding to that of the second web of material (11).

2. Device as defined in Claim 1, characterized in that the first curved section (a) serves to guide the conveying elements (1) during takeover of the second web of material (11) and its connection with the tags of the first web of material (9) and extends over an angular area of at least 150 degrees to approximately 180 degrees.

3. Device as defined in Claims 1 or 2, characterized in that the neighbouring conveying elements are spaced at a minimal distance during separation of the tags from the first web of material by a knife (10), with the front conveying element (1) in terms of the direction of rotation being guided on the curved section (c) and the neighbouring conveying element (1) following it being guided on the first curved section (a) for a predeterminable equal period of time.

4. Device as defined in one of Claims 1 to 3, wherein the beginning and the end of the receiving surface of a conveying element (1) in terms of its circumference are each engaged by a clamping device (12) which is closed or open in certain angular positions of the clamping elements (1), characterized in that the clamping device (12) is an angular lever (13) which can swivel about an axis parallel to the circular arc-shaped receiving surface of the conveying elements, with one of its legs being adapted to be pressed against the receiving surface of the conveying element (1) and its other leg to be alternately swivelled into the open and closed positions by guidance via a control pin (17) on a cam plate (15).

5. Use of a device as defined in one of Claims 1 to 4 for manufacturing disposable diapers consisting of sections of web material with leg end regions extending in the longitudinal direction of the sections of web material and covering only part of the length between the ends of the sections of web material.

**Revendications**

1. Dispositif pour appliquer des morceaux de ruban étirés d'une première bande élastique (9) sur des zones séparées, se trouvant l'une derrière l'autre à une distance prédéterminée, d'une seconde bande (11) se déplaçant continuellement, dans lequel une pluralité d'éléments de transport (1) ayant, à chaque fois, une surface de réception rotative, se trouvant sur une même circonférence et pouvant s'appliquer sur les deux bandes situées l'une derrière l'autre, tournent sur un axe commun, et dans lequel les éléments de transport (1) présentent l'un par rapport à l'autre un écartement circonférentiel, et, à chaque tour, leur vitesse angulaire varie de façon prédéfinie, de sorte que, dans une première position prédéfinie de façon fixe, dans laquelle la première bande (9) est appliquée sur les surfaces de réception des éléments de transport (1), à chaque fois deux éléments de transport se trouvent l'un derrière l'autre à un écartement minimal, l'élément de transport avant dans le sens de déplacement, ayant une vitesse angulaire plus grande que l'élément de transport (1) voisin suivant, s'éloignant par augmentation de l'écartement, et, après avoir atteint un écartement, pour lequel les morceaux de ruban entraînés doivent être appliqués sur la seconde bande (11), transmettant sur la seconde bande (11) les morceaux de ruban dans une seconde position prédéfinie de façon fixe, caractérisé en ce que :

a) sur l'axe des éléments de transport (1), tourne un disque d'entraînement (2),

b) sur un secteur du disque d'entraînement (2) pour chaque élément de transport (1), est monté rotatif un premier levier (3) sur un axe (5) parallèle à l'axe commun des éléments de transport (1),

c) l'extrémité radialement interne de chaque premier levier (3) peut être guidée dans une voie incurvée prédéfinie (8),

d) à chaque extrémité radialement externe de chaque premier levier, est articulé un second levier (4),

e) les seconds leviers (4) sont reliés, de façon articulée, à un élément de transport respectif (1),

f) les points d'articulation (6) pour les seconds leviers (4) se trouvent au même endroit sur chaque élément de transport (1),

g) la voie incurvée (8) est divisée en quatre tronçons consécutifs (a, b, c, d) desquels deux tronçons généralement opposés (a, c) sont des arcs de cercle concentriques à l'axe commun des éléments de transport (1), un tronçon étant réalisé sous forme d'une voie incurvée (b) ayant un rayon croissant par rapport à l'axe commun et un tronçon étant réalisé sous forme d'une voie incurvée (d) ayant un rayon décroissant par rapport à l'axe commun,

h) le premier tronçon incurvé (a), dans lequel a lieu le guidage des éléments de transport (1) pendant la réception de la seconde bande (11) et sa liaison aux morceaux de ruban de la première bande (9), s'étend sur une zone angulaire, qui est choisie de sorte que chaque élément de transport est déplacé à une vitesse angulaire constante sur une distance correspondant au moins à la longueur des morceaux de ruban, pour appliquer un morceau de ruban sur la seconde bande (11), la vitesse des éléments de transport (1) coïncidant avec celle de la seconde bande (11).

2. Dispositif selon la revendication 1, caractérisé en ce que le premier tronçon incurvé (a) sert au guidage des éléments de transport (1) pendant la réception de la seconde bande (11) et sa liaison aux morceaux de ruban de la première bande (9) et s'étend sur une zone angulaire d'au moins 150° à environ 180°.

3. Dispositif selon la revendication 1 ou 2, caractérisé en ce que, pendant la séparation des morceaux de ruban de la première bande par un couteau (10), les éléments de transport voisins présentent un écartement minimal, par quoi, pendant un même intervalle de temps prédéterminable, l'élément de transport (1) avant dans le sens de rotation est guidé sur le tronçon incurvé (c) et l'élément de transport (1) voisin suivant est guidé sur le premier tronçon incurvé (a).

4. Dispositif selon une des revendications 1 à 3, dans lequel, au début et à la fin circonférentiels de la surface de réception d'un élément de transport (1), agit à chaque fois, un dispositif de serrage (12), qui est ouvert ou fermé pour des positions angulaires déterminées des éléments de transport (1), caractérisé en ce que le dispositif de serrage (12) est un levier coudé (13) pouvant pivoter autour d'un axe parallèle à la surface de réception en forme d'arc de cercle des éléments de transport, dont une branche peut être pressée contre la surface de réception de l'élément de transport (1) et dont l'autre branche peut pivoter alternativement dans la position d'ouverture et la position de fermeture par guidage, par l'intermédiaire d'une broche de commande (17), contre une came (15).

5. Utilisation d'un dispositif selon une des revendications 1 à 4 pour réaliser des couches jetables constituées de morceaux de bande, ayant des zones d'isolation des jambes, s'étendant en direction longitudinale des morceaux de bande, qui n'embrassent qu'une longueur partielle entre les extrémités des morceaux de bande.

# Fig. 1

EP 0 141 338 B1

**Fig. 2**

**Fig. 3**

2